# EUROPÄISCHE PATENTSCHRIFT

(11) **EP 1 533 036 B1**
(45) Veröffentlichungstag und Bekanntmachung des Hinweises auf die Patenterteilung: **09.06.2010**
(21) Anmeldenummer: 04090439.3
(22) Anmeldetag: 17.11.2004
(51) Int. Cl.: B01L 3/00, G02B 21/36, G03B 21/00

(54) **Vorrichtungskombination umfassend Probenträger und Lesegerät**
Combined apparatus comprising a sample holder and a reading device
Appareil combiné comprenant un porte-échantillon et un lecteur

(30) Priorität: 18.11.2003 DE 20317944 U
(43) Veröffentlichungstag der Anmeldung: 25.05.2005
(73) Patentinhaber: Heiser, Volker, Dr., 10999 Berlin (DE); Landt, Olfert, 10965 Berlin (DE)
(72) Erfinder: Heiser, Volker, Dr., 10999 Berlin (DE); Landt, Olfert, 10965 Berlin (DE)
(74) Vertreter: Junghans, Claas

(56) Entgegenhaltungen:
- DE-A1- 10 038 080
- US-A- 3 780 298
- US-A- 5 784 162
- US-A1- 2002 058 331

## Beschreibung

### Gebiet der Erfindung

Die vorliegende Erfindung betrifft das Gebiet der medizinischen und biologischen Diagnose. Im Speziellen betrifft die Erfindung das Gebiet der Biochips zur molekularen Diagnostik.

### Hintergrund der Erfindung

Die sogenannte Chip-Technologie ist in den letzten Jahren ein wichtiges Gebiet der biologischen Technologieentwicklung geworden. Unter Chip-Technologie sei im Folgenden die Vielzahl von Methoden verstanden, die darauf beruhen, biologische Moleküle wie Nukleinsäuren, Peptide und Proteine oder organische Moleküle, welche mit biologisch relevanten Molekülen wechselwirken, an eine Oberfläche zu binden und die Interaktion dieser gebundenen Moleküle mit einer Probe zu untersuchen. Diese Anordnung ermöglicht die parallele Untersuchung einer Probe mit einer großen Zahl verschiedener Reaktanden.

Aktuelle Zusammenfassungen des Standes der Wissenschaft auf diesem Gebiet sind z.B. die von Ng und llag (Biotechnol Annu Rev. 2003; 9:1-149) oder Jain (Curr Opin Drug Discov Devel. 2004, 7(3):285-289) und die darin enthaltenen Literaturverweise.

Am bekanntesten ist die Bindung von Nukleinsäuren an Oberflächen, welche zu "DNA-Chips" führt. Solche zweidimensionale Anordnungen ("Arrays") von Nukleinsäuren können zur Analyse einer Vielzahl oder gar der Gesamtheit der in einer Zelle oder einem Gewebe vorhandenen Gene (des Genoms) oder der dort hergestellten Transkripte (des "Transkriptoms") verwendet werden. Mit der zunehmenden Fokussierung vieler wissenschaftlicher Fragestellungen auf die parallele Untersuchung von immer mehr Parametern hat sich die Array-Technologie hin zu komplexeren Chips mit immer mehr der untersuchten Probe angebotenen Bindungspartnem entwickelt. Gleiches gilt neben den DNA-Arrays auch für Protein- und Peptidchips.

Mit der Entwicklung von Arrays immer höherer Dichte und Komplexität hat sich deren Leistungsfähigkeit enorm gesteigert. Gleichzeitig sind allerdings auch die Kosten für die Herstellung und Analyse dieser Hochdichte-Arrays gestiegen.

Während die Forschung weiterhin Biochips hoher Dichte nachfragt, werden parallel immer mehr Anwendungen der Array-Technologie für Fragestellungen der Routineuntersuchung entwickelt. Zu diesen gehören z.B. Fragestellungen der Mikrobiologie (Bestimmung und Typisierung von Erregern oder Resistenzen gegen Antibiotika), aus dem Bereich der Lebensmitteltechnik oder Forensik. Diese bedürfen in vielen Fällen keiner hochdichten Arrays von tausenden immobilisierter Liganden, sondern typischerweise unter hundert verschiedene gebundene Reaktionspartner. Vielmehr stehen bei Routineanwendungen Aspekte der Reproduzierbarkeit, Einfachheit der Analysendurchführung und vor allem die Gesamtkosten der Analyse im Vordergrund. Im Hinblick auf den Kostenaspekt sind dabei nicht nur die Kosten für den Chip und die zu seiner Bearbeitung notwendigen Verbrauchsmaterialien, sondern auch die Kosten der Instrumentierung zur Analyse zu berücksichtigen. Viele Lesegeräte für Arrays sind hochkomplexe und sehr teure Anlageinvestitionen, deren Anschaffung nur in Ausnahmefällen durch eine kommerzielle Nutzung zur Routineanalyse getragen werden kann.

Die wesentlichen notwendigen Technologiekomponenten für die Herstellung und Prozessierung von Biöchips sind bekannt:

Als Trägermaterialien für die zu bindenden Biomoleküle sind z. B. Glas, thermoplastische Polymere, Silizium, mit Gold bedampftes Glas oder Aluminium bekannt.

Zur Bindung der zu immobilisierenden Biomoleküle an die Oberfläche der Probenträger werden verschiedene Verfahren genutzt. Zum einen kann die Bindung durch Adsorption an die Trägeroberfläche erfolgen. Die Biomoleküle können aber auch kovalent an entsprechend aktivierte Oberflächen gebunden werden. Auch die Biomoleküle können aktiviert werden, damit sie kovalent an die Oberfläche binden. Darüber hinaus wurden spezielle Beschichtungen entwickelt, die eine hohe und reproduzierbare Anbindung der Biomoleküle ermöglichen sollen.

Die Detektion eines Bindungsereignisses kann auf verschiedene Weise erfolgen. Für die Detektion der Bindung von Nukleinsäuren, welche in der Regel über die sequenzspezifische Wechselwirkung von hybridisierungsfähigen Nukleinsäuren oder deren Analoga erfolgt, ist die verbreiteteste Detektionsmethode die Fluoreszensdetektion von gebundenen Probenmolekülen, welche vor der Bindung mit Fluoreszenzfarbstoffen direkt markiert worden waren, oder vor bzw. nach der Bindung an den Array mit einem dritten Reagenz detektiert werden (siehe ebenfalls Jain, ibid.). Ebenfalls kann die Detektion enzymatisch oder radioaktiv erfolgen, bei Verwendung eines entsprechend ausgestatteten Probenträgers ist weiterhin die direkte Messung eines veränderten physikalischen Parameters wie Plasmon-Oberflächenresonanz oder Leitfähigkeit möglich.

Die Detektion von Fluoreszenzveränderungen erfordert einen relativ großen apparativen Aufwand. Taton et al. (Science 289, 1157-1161 (2000); US 6,361,944) beschreiben die Detektion von Nukleinsäuren, welche mit Gold-Nanopartikeln gekoppelt sind. Die Anwesenheit gebundener Probenmoleküle wird durch die nachfolgende Reduktion von Silber (I)-lonen zu metallischem Silber bei Licht im sichtbarem Bereich erkennbar und durch konventionelle Dokumentenscanner densitometrisch auswertbar gemacht. Die Autoren sprechen von einer Empfindlichkeitssteigerung von mindestens zwei Größenordnungen gegenüber gleichartigen Fluoreszenzdetektionsmethoden.

In diesen wie den meisten anderen Anwendungen der Array-Technologie ist das Format, auf dem der Array hergestellt und in Verkehr gebracht wird, der konventionelle gläserne Objektträger für die Mikroskopie. Die Abmessungen eines Objektträgers sind 7,5 x 2,5 cm oder 1 x 3 inch und einer Stärke von 0,5 bis 1,5 mm. Glas war historisch meist der Ausgangspunkt für die Kopplungschemie zur Bindung der auf den Array zu immobilisierenden Biomoleküle. Dazu kommt, dass mit der zunehmenden Dichte der Arrays die einzelnen Punkte, die einen speziellen Liganden repräsentierten, immer kleiner werden mussten und deshalb die Detektion mit einem mikroskopischen Verfahren naheliegt. Dies fördert weiter den Trend zu technisch komplexen, teuren Detektionsgeräten. Für hochdichte Chips werden üblicherweise die Ergebnisse einer Analyse mit einem konfokalen Mikroskop ausgelesen. Die Kosten belaufen sich dabei auf 25.000,- bis 150.000,- Euro pro Gerät (Stand 2003).

Die einzige bekannte Ausnahme ist das Auslesen von konventionellen Mikroskopie-Objektträgern mit Durchlicht-Flachbett-Scannern von Nanosphere. Inc. oder von Arraylt Inc.. Die Handhabung von Objektträgern bringt aber weiterhin Nachteile hinsichtlich der Automatisierbarkeit der Herstellung und Prozessierung mit sich, die eine Verbesserung der Technologie wünschenswert erscheinen lassen. Insbesondere ist die Auflösung kommerzieller Flachbettscanner zu gering, um quantitative Daten auf Biomolekül-Arrays zu erfassen. Weiterhin muss ein auf konventionellen Objektträgern angebrachter Array in der richtigen Orientierung eingelegt und präzise fixiert werden, um das entstehende Bild -ungeachtet der unzureichenden Auflösung- automatisch auslesbar zu machen.

Geeignete Materialien, Kopplungsmethoden für Biomoleküle sowie Anwendungsbeispiele sind u.a. in der DE 1032104 offenbart.

Ausgehend von diesem Stand der Technik ist es Aufgabe der vorliegenden Erfindung, eine Kombination aus Chip und Detektionsapparatur zur Verfügung zu stellen, welche eine wirtschaftliche Herstellung und Prozessierung von Arrays aus Biomolekülen zu Zwecken der Routineanalyse erlaubt.

Erfindungsgemäß wird diese Aufgabe gelöst, indem zur Herstellung der Biochips ein Probenträgerformat gewählt wird, welches dem konventioneller Kleinbildfilm-Diapositive entspricht. Weiterhin ist ein wesentliches Element der erfinderischen Lösung die Verwendung von sog. Diapositiv-Scannern bzw. Lesegeräten, welche die auf dem Array im Diapositiv-Format enthaltene optische Information digitalisieren und damit der Computerauswertung zugänglich machen.

Ein wesentlicher Vorteil dieser Erfindung ist es, dass damit der erfinderische Probenträger im Diapositiv-Format (50 mm x 50 mm) des Arrays in automatisierbarer Form über im Fachhandel erhältliche Magazine eingelesen und gehandhabt werden kann. Zudem kann eine breite Palette an Zubehörgegenständen für die Archivierung, die Reinigung und den Transport der Probenträger eingesetzt werden. Des weiteren kann die Projektion der Ergebnisse mit handelsüblichen Dia-Projektoren erfolgen.

Ein wesentlicher weiterer Vorteil der Erfindung ist, dass die handelsüblichen Lesegeräte, also Diascanner und elektronische Kameras mit Halterungseinrichtungen für Kleinbildfilm-Diapositive eine gegenüber Ftachbett-Scannern sehr viel höhere Auflösung und präzisere Ausleuchtung aufweisen, die das Auslesen auch quantitativer Analyseergebnisse von mit Silberreduktion entwickelten bzw. anderen Färbemethoden gefärbten Arrays erlaubt.

Ein weiterer Vorteil der Erfindung ist, dass gegenüber verschiebbaren Masken oder manuel auf Flachbettscannern positionierten Objektträgern die automatisierbare Handhabung und Zehntelmilimeter-genaue Positionierung von Diapositivformatierten Probenträgern ein reproduzierbares automatisches Auslesen von Routineanalysen erlaubt.

Ein weiterer Vorteil ergibt sich aus einer bevorzugten Ausführung des Trägers als Kunststoffteil mit oder ohne aufgebrachte Strukturen. Der Probenträger kann durch die Verwendung von Kunststoffen, wie Polyethylen, Polypropylen, Polyether, Polyetherimid, Polystryrol, Polycarbonat, Polysulfon, Polyethersulfon, Polyvinylidenfluorid, PTFE, Polyamid, Polyimid oder anderen Kunststoffen, besonders kostengünstig und strapazierfähig hergestellt werden.

Weiterhin ist vorteilhafterweise bei entsprechender Ausgestaltung der Probenträger eine Entwicklung oder Prozessierung derselben in Maschinen der Photobearbeitung möglich, was wiederum aufwendige apparative Entwicklungen vermeidet und den Gesamtprozess zeitlich und finanziell erheblich erleichtert.

Weitere Vorteile folgen aus der Ausgestaltung des Probenträgers mit einem Funktionsteil, welches aus in Reihen und Spalten angeordneten Funktionszonen besteht. Dies ermöglicht die parallele Prozessierung und Auslesung verschiedener Proben. Die Anordnung mehrerer Proben auf einem Träger führt zudem zu einer Kostenreduzierung der Probenanalyse.

Weiterhin kann erfindungsgemäß die Handhabung von konventionellen Objektträgern erleichtert werden, indem ein dem Diapositiv-Format 50 x 50 mm entsprechender Adapter in Kombination mit Leseeinrichtungen für Diapositive zur Analyse von Chips im Objektträger-Format verwendet wird.

### Detaillierte Beschreibung der Erfindung

Die bevorzugten Ausführungsbeispiele des Probenträgers können in drei Gruppen zusammengefasst werden. Die erste Gruppe umfasst Ausführungsbeispiele des Probenträgers ohne Rahmenteil, wobei im Falle mehrerer Funktionszonen, diese von randbildenden Erhebungen umgeben sind. Die zweite Gruppe umfasst Ausführungsbeispiele des Probenträgers mit Rahmenteil, wobei im Falle mehrerer Funktionszonen, diese von randbildenden Erhebungen umgeben sind. Die dritte Gruppe umfasst Ausführungsbeispiele des Probenträgers mit Rahmenteil, wobei die Funktionszonen vertieft ausgebildet sind.

Der erfindungsgemäße Probenträger weist bevorzugt eine quadratische Grundfläche von 50 x 50 mm (+/-2 mm) und eine Stärke (Dicke) von maximal 2mm (+0,5mm/- 1mm) auf. Die gesamte Beschaffenheit der Oberfläche kann dabei einheitlich sein, oder ist in ein zentral gelegenes Funktionsteil mit rechteckigen Abmessungen von maximal 36 x 24 mm (+/- 1 mm) und eine äußeres Rahmenteil unterteilt. Das Funktionsteil besteht in bevorzugter Ausgestaltung aus mehreren Funktionszonen. In einer weiteren bevorzugten Ausgestaltung nimmt das Funktionsteil die gesamte Probenträgeroberfläche ein.

Die Ausgestaltung des Funktionsteils oder der gesamten Oberfläche ist dabei bevorzugt hochtransparent-farblos, um eine maximale Durchleuchtung des Probenträgers zu ermöglichen.

In einer weiteren bevorzugten Ausgestaltung ist die Oberfläche des Funktionsteils oder des gesamten Trägers hochtransparent und eingefärbt, um nur Licht einer bestimmten Wellenlänge passieren zu lassen. Hier dient die hochtransparent eingefärbte Oberfläche als Filter.

In einem weiteren bevorzugten Ausführungsbeispiel ist die Oberfläche des Funktionsteils oder des gesamten Trägers semitransparent bzw. milchig ausgebildet. Dies gewährleistet bei einer punktförmigen Lichtquelle eine möglichst gleichmäßige Ausleuchtung.

In weiterer bevorzugter Ausgestaltung ist die Oberfläche des Funktionsteils oder des gesamten Trägers lichtundurchlässig ausgebildet. Dies ermöglicht Auslesungen im Auflicht, z. B. für Reflektion und Fluoreszenz, oder aber von der Lichtquelle unabhängige Ausleseverfahren wie Chemo- bzw. Biolumineszenz.

Das Funktionsteil wird bevorzugt durch verschiedene runde oder eckige Erhebungen oder Vertiefungen in einzelne Funktionszonen unterteilt. Diese Erhebungen oder Vertiefungen begünstigen die individuelle Benetzung, Überschichtung oder Inkubation der Funktionszonen mit Flüssigkeiten. Die randbildenden Erhebungen sind bevorzugt hydrophob beschichtet und minimieren durch ihre Ausgestaltung den Überlauf eingebrachter Flüssigkeiten. Im Falle von Vertiefungen minimieren die außerhalb der Funktionszonen gelegenen höheren Bereiche durch ihre Ausgestaltung den Überlauf eingebrachter Flüssigkeiten.

Die regelmäßige Anordnung der Funktionszonen folgt vorzugsweise den etablierten Mikrotiterplattenformaten, wie dem 24-Loch-Format, bei dem der Zentrum-zu-Zentrum Abstand 18 mm beträgt, dem 96-Loch-Format mit einem Zentrum-zu-Zentrum Abstand von 9 mm oder dem 384-Loch-Format mit einem Zentrum-zu-Zentrum Abstand von 4,5 mm. Die Funktionszonen sind bevorzugt rund, eckig oder linienförmig ausgestaltet.

Die Oberfläche der Funktionszonen oder die gesamte Oberfläche des Trägers wird bevorzugt chemisch modifiziert. Die chemische Modifikation besteht dabei aus freien Amino- oder Aldehyd- oder Carboxy- oder Epoxy- oder Hydroxy- oder Sulfhydrylgruppen oder Gemischen aus allen Kombinationen dieser Gruppen. Die Modifikation ermöglicht die Immobilisierung oder Anbindung von Molekülen in Form einer flächigen Beschichtung oder in Rastern aus Funktionszonen, den Arrays. Die Oberflächen werden bevorzugt mit einem Gel beschichtet, so dass es sich, in mikroskopischen Maßstäben betrachtet, um dreidimensionale Träger handelt.

In einer bevorzugten Ausgestaltung des Probenträgers werden synthetische DNA Oligonukleotide oder Produkte der Polymerase Kettenreaktion (PCR) oder der Ligasekettenreaktion (LCR) zur Analyse von Nukleinsäuren kovalent oder nichtkovalent an der Oberfläche des Probenträgers gebunden. Dazu wird zunächst im einem ersten Schritt die Oberfläche des Probenträgers in einer geeigneten Weise aktiviert. Dann werden die Oligonukleotide aus einer Lösung oder Suspension mittels eines Stempels, mit einer Nadel, mit einer Pipettenspitze oder kontaktfrei in einem Sprühverfahren auf die Oberfläche aufgebracht. Alternativ werden diese Moleküle durch Bestrahlung oder durch Wärmebehandlung angebunden. Eine weitere Möglichkeit der Bindung ist zudem die rein adhaesive Bindung aufgrund elektrostatischer Kräfte. Die gebundenen Oligonukleotide entsprechen dabei jenen Nukleinsäuren, die in der Folge nachgewiesen werden sollen.

Die nachzuweisenden Nukleinsäuren stellen typischerweise doppelsträngige Sequenzen dar, die mittels der PCR erzeugt wurden, oder aber Boten-Ribonukleinsäure oder isolierte DNA. Die nachzuweisende Nukleinsäure wird zunächst mit einem Fluoreszenzfarbstoff, mit Biotin, mit Partikeln oder mit einem Hapten entweder kovalent oder durch Bindung eines weiteren, modifizierten Oligonukleotides markiert. Eine bevorzugte Markierung für PCR-Produkte erfolgt durch die Verwendung entsprechender Oligonukleotidprimer. Die nachzuweisende Nukleinsäure wird durch Erhitzen denaturiert und in einer geeigneten Pufferlösung auf die Oberfläche gegeben, so dass sich Bindungen zwischen komplementären Sequenzen ausbilden. Anschließend werden nicht gebundene Nukleinsäuren durch Waschen entfernt. Die Bindung wird entweder über den Nachweis des Fluoreszenzfarbstoffes, durch Detektion der Partikel oder durch eine Bindungsreaktion an das Hapten nachgewiesen. Die Detektion kann mit oder ohne eine nachfolgende Verstärkung erfolgen. Bevorzugte Nachweisverfahren sind die Bindung enzymkonjugierter Antikörper, die das Hapten binden, konjugiertes Streptavidin, das Biotin bindet, und nachfolgender enzymatischer Reaktion, bei der ein Farbstoff freigesetzt wird, oder aber die Bindung von Nanopartikeln, welche über eine Färbung nachgewiesen werden kann. Als Material der Nanopartikel wird bevorzugt Nanogold, das als Kristallisationskeim für die Reduktion von Silber oder Gold dienen kann, eingesetzt. Für eine optische Auswertung werden bevorzugt Systeme, die einen dunklen Farbstoff entwickeln, dunkle Partikel verwenden oder aber auf der Ausscheidung elementaren Silbers beruhen, eingesetzt.

### Beispiel

### Streptavidhi-Beschlchtuna eines DNA-Arrays

Ein erfindungsgemäßer Kunststoffträger mit den äußeren Abmessungen 50 x 50 mm wird mit Streptavidin beschichtet, indem er für 2 h mit einer Lösung von 10 µg/ml Streptavidin in PBS Puffer (10 mM Phosphat, 3 mM Kaliumchlorid, 137 mM Natriumchlorid) überschichtet wird.

Der Träger wird zwei mal 1 min mit PBS und einmal in destilliertem Wasser gewaschen und getrocknet. Weitere Bindungsstellen werden durch 1 h Inkubation mit Blockierungsreagenz (Roche Diagnostics, nach Anleitung des Herstellers) oder Milchpulverlösung in Wasser (3%) abgesättigt. Der Träger wird drei mal in destilliertem Wasser gewaschen und getrocknet.

### Aufbringung spezifischer Sonden über Biotin-Streptavidin

5'-terminal biotinylierte DNA Oligonukleotidsonden werden aus einer 20 µM Lösung mittels eines Stempels punktförmig auf die Oberfläche aufgebracht. Der Stempel hat einen Durchmesser von vorzugsweise 0,05-0,3 mm Nach dem Auftrocknen der Proben werden die Kunststoffträger zwei mal für eine Minute in PBS Puffer mit 0,05% Tween 20 und zwei mal in Wasser gewaschen und getrocknet. Freies Streptavidin auf den Kunststoffträgern wird mit einer Lösung von biotinyliertem Oligo-dT abgesättigt, indem sie für 10 min mit einer 0,2 mM Lösung von Biotin in PBS beschichtet und wie oben gewaschen werden.

Für die Herstellung eines DNA-Arrays für die Identifizierung von Spezies in Fleischwaren wurden die Sequenzen SEQ ID NO 01: 5'-Biotin-TAACAACAATCTCCATgAgTTggT aus der 16S RNA Region vom Rind, SEQ ID NO 02: 5'-Biotin-gATAAAACATAACTTAACATggAC aus der Sequenz vom Schwein und SEQ ID NO 03: 5'-BiotingACCACCTTACAACCTTACACAgC aus der Sequenz vom Huhn an drei verschiedene Punkte auf dem Kunststoffträger aufgetragen und wie oben behandelt.

### Hybridsierung von PCR-Produkten

Proben von verschiedenen Fleischwaren werden 10 min mit 0,8% NaCl Lösung geschüttelt und abzentrifugiert. Von dem Überstand werden 2 µl Volumen mit je 10 pmol der PCR-Primer SEQ ID NO 04: 5'-Fluorescein-TgATCCAACATCgAggTCgTAAACC und SEQ ID NO 05: 5'-AAgACgAgAAgACCCTRTggARCT sowie Nukleotiden, Puffer und Taq Polymerase (aus dem Roche FastStart Reagenz) versehen und auf ein Volumen von 20 µl gebracht. Es werden 45 Thermozyklen mit 5 s bei 95°C, 10 s bei 60°C und 5 s bei 72°C durchgeführt. Das PCR Produkt wird mit 10 µl 20x SSC (3 M Natriumchlorid, Natriumcitrat 0,3 M, pH 7,0) Puffer und 3 µl 5% SDS versetzt, für 10 sec gemischt und auf den trockenen DNA-Array gegeben und für 30 min bei 50°C inkubiert. Der Kunststoffträger wird zwei mal 1 min in 0,2 x SSC Puffer gewaschen und getrocknet.

### Immunologische Detektion der Bindung

Zur Detektion wird 20 µl einer Lösung von 1 µg/L Anti-Fluorescein-Meerrettich Peroxidase Konjugat (Seramun) auf den Kunststoffträger gegeben und für 10 min inkubiert.

Der Kunststoffträger wird drei mal 1 min in 0,2 x SSC Puffer gewaschen und getrocknet. Zum Nachweis der Bindung wird 20 µl einer Lösung von TMB (3,3',5,5'-Tetramethylbenzidin) (Seramun) auf den Kunststoffträger gegeben und für 5-10 min inkubiert. Die Reaktion wird durch Waschen mit Wasser beendet.

### Auslesen der Resultate

Die Oxidation des TMB führt zur ortsgebundenen Präzipitation eines blauen Niederschlages an den Positionen, an denen Peroxidase Aktivität vorhanden ist. Mit handelsüblichen Diascannern (z.b. Minolta Dimage Scan 5400, Nikon Supercoolscan 8000 LD etc.) können die Signale auf dem erfindungsgemäßen Probenträger aufgrund seiner äusseren Abmessungen (50 x 50 mm) und der relativen Lage der Reaktionszonen (zentral 36 x 24 mm) im Durchlicht-Scanbetrieb in Bilddateien verschiedener Formate umgewandelt werden, vorzugsweise in TIF-Dateien im 16-bit Graustufenmodus. Herkömmliche Bildanalyse-Programme für die Auswertung biologischer Array-Systeme (z.b. GenePix Pro, Axon Instruments, USA; SlideReader, Chipron GmbH, Deutschland) können diese Bilddateien auslesen und in Verbindung mit Zuordnungsdateien, welche die Informationen über die relative Lage der spezifischen Fängermoleküle in den Reaktionszonen bereitstellen, eine automatische Auswertung vornehmen. Es kann sowohl eine reine Ja/Nein Interpretation erfolgen, bei der die nachweisbare Bildung des blauen Niederschlages das Vorhandensein des jeweiligen Analyten in der Probe bedeutet (Rind-Fängermolekül belegt Anwesenheit von Rinder-DNA), als auch eine quantitative Auswertung erfolgen, da die vom Diascanner in die Bilddatei übertragene Signalintensität mit der vorhandenen Menge des Anälyten in der Probe korreliert ist.

### SEQUENCE LISTING

<110> Heiser, Volker
   Landt, Olfert
<120> Vorrichtungskombination umfassend Probenträger und Lesegerät
<130> CHIP.EPA.2004.1
<140> 04090439.3-****/
   <141> 2004-11-17
<150> DE20317944U1
   <151> 2003-11-18
<160> 5
<170> PatentIn version 3.1
<210> 1
   <211> 24
   <212> DNA
   <213> Bos taurus
<400> 1
   taacaacaat ctccatgagt tggt 24
<210> 2
   <211> 24
   <212> DNA
   <213> Sus scrofa
<400> 2
   gataaaacat aacttaacat ggac 24
<210> 3
   <211> 24
   <212> DNA
   <213> Gallus gallus
<400> 3
   gaccacctta caaccttaca cagc 24
<210> 4
   <211> 25
   <212> DNA
   <213> Sus scrofa
<400> 4
   tgatccaaca tcgaggtcgt aaacc 25
<210> 5
   <211> 24
   <212> DNA
   <213> Sus scrofa
<400> 5
   aagacgagaa gaccctrtgg arct 24

## Patentansprüche

1. Verfahren zur Detektion von Biomolekülen umfassend
a) das Aufbringen von einer oder mehrerer Mengen Bindungspartner der Biomoleküle auf eine im wesentlichen ebene Oberfläche vermittels kovalenter oder quasi-kovalenter Bindung, ionische Wechselwirkungen, Adhaesion oder Adsorption, die Entfernung nicht an die Oberfläche gebundener Bindungspartner und die nachfolgende Aufbringung einer Biomoleküle enthaltenen Probe auf die mit Bindungspartnern beschichtete Oberfläche unter Bedingungen, die die Bindung von Biomolekülen an ihre Bindungspartner erlauben, und nachfolgendes Abwaschen nicht an Bindungspartner gebundener Biomoleküle und anderer Probenbestandteile;
b) die Kenntlichmachung der gebundenen Biomoleküle durch eine im sichtbaren Wellenlängenbereich detektierbare Farbreaktion und
c) die Erfassung der gebundenen Biomoleküle über eine Abbildung in einem optischen Detektionssystem,
**dadurch gekennzeichnet dass**
die im wesentlichen ebene Oberfläche in einem Rahmen der Abmessungen eines herkömmlichen Kleinbildfilm-Diapositivrahmens von 50 mm mal 50 mm angeordnet ist und die Erfassung der gebundenen Biomoleküle durch die Optik eines Diapositivscanners mit elektronischer Bilderfassung erfolgt.

2. Verfahren gemäß Anspruch 1, wobei die Kenntlichmachung der gebundenen Biomoleküle durch eine im sichtbaren Wellenlängenbereich detektierbare Farbreaktion **dadurch** erfolgt, dass entweder
a) in einem ersten Schritt ein zur Umsetzung eines Farbstoffes geeignetes Enzym, welches eine für gebundene Biomoleküle spezifische Bindungseigenschaft hat, mit der Oberfläche in Kontakt gebracht wird, und in einem zweiten Schritt eine Lösung eines Farbstoffes, der von dem Enzym umgesetzt werden kann, mit der Oberfläche in Kontakt gebracht wird, so dass an den Orten, an denen Biomoleküle mit dem an ihnen gebundenen Enzym an Bindungpartner gebunden haben, eine Farbreaktion entsteht, oder dass
b) ein mit dem Biomolekül verbundener fluoreszens- oder lumineszenzaktiver Farbstoff durch geeignete optische oder chemische Aktivierung zur Emission von Licht gebracht wird, oder dass
c) ein mit dem Biomolekül verbundenes Metallpartikel als Kristallisationskeim für eine nachfolgende Behandlung mit einem Edelmetallsalz, vorzugsweise einem Silbersalz, unter reduzierenden Bedingungen verwendet wird, so dass ein im sichtbaren Licht sichtbarer Metallniederschlag entsteht.

3. Vorrichtung umfassend
a) einen Probenträger für Biomoleküle, umfassend eine im wesentlichen ebene Oberfläche, an die Bindungspartner für Biomoleküle vermittels kovalenter, quasi-kovalenter, ionischer, adhaesiver oder adsorbtiver Wechselwirkungen gebunden sind, wobei der Probenträger in einem Rahmen der Abmessungen eines herkömmlichen Kleinbildfilm-Diapositivrahmens von 50 mm mal 50 mm angeordnet ist, sowie
b) einen Diascanner, der zur elektronischen Detektion des Bildes der Oberfläche eines in ihn eingebrachten Probenträgers im sichtbaren Licht in der Lage ist.

4. Probenträger für Biomoleküle, umfassend eine im wesentlichen ebene Oberfläche, an die Bindungspartner für Biomoleküle vermittels kovalenter, quasi-kovalenter, ionischer, adhaesiver oder adsorbtiver Wechselwirkungen gebunden sind, wobei der Probenträger in einem Rahmen der Abmessungen eines herkömmlichen Kleinbildfilm-Diapositivrahmens von 50 mm mal 50 mm angeordnet ist.

## Claims

1. Method for the detection of biomolecules, comprising:
a) applying one or several quantities of binding partners of the biomolecules onto a substantially planar surface by means of covalent or quasi-covalent binding, ionic interaction, adhesion or adsorption, removing binding partners not attached to the surface and subsequently applying a sample containing biomolecules onto the the surface covered with binding partners under conditions which allow the binding of biomolecules to their binding partners, and subsequent washing off of biomolecules not bound to binding partners and other sample constituents,
b) labelling the bound biomolecules by a colour reaction detectable at visible wavelength range and
c) detecting the bound biomolecules by an image in an optical detection system, **characterized in that**
the substantially planar surface is provided in a frame having the measure of a customary diapositive film frame of 50 mm by 50 mm and the detection of the bound biomolecules is effected by the lenses of a diapositive scanner by electronic image capture.

2. Method according to claim 1, whereby the labelling of the bound biomolecules is achieved by a colour reaction detectable at visible wavelength range in such fashion that either
a) in a first step, an enzyme having a binding capacity specific for bound biomolecules and being suited for reacting a dye compound, is contacted with the surface, and in a second step, a solution of a dye that can be reacted by the enzyme, is contacted with the surface, so that at locations where biomolecules have bound to binding partners, with enzymes attached to them, a colour reaction develops, or that
b) a fluorescent or luminescent dye attached to the biomolecule is induced to emit light by suitable optical or chemical activation, or that
c) a metal particle attached to the biomolecule is used as a cristallization seed for subsequent treatment with a salt of a noble metal, preferably a silver salt, under reducing conditions, providing a metal precipitate visible in visible light.

3. Device, comprising
a) a sample holder for biomolecules, comprising a substantially planar surface, onto which binding partners for biomolecules are attached by means of covalent or quasi-covalent, ionic, adhesive or adsorptive interaction, whereby the sample holder provided in a frame having the measure of a customary diapositive film frame of 50 mm by 50 mm, and
b) a diapositive scanner suited to electronically detect the image of the surface of a sample holder mounted therein in visible light.

4. Sample holder for biomolecules, comprising a substantially planar, onto which binding partners for biomolecules are attached by means of covalent or quasi-covalent, ionic, adhesive or adsorptive interaction, whereby the sample holder provided in a frame having the measure of a customary diapositive film frame of 50 mm by 50 mm.

## Revendications

1. Procédé de détection de biomolécules comportant les étapes de :
(a) l'application d'une ou plusieurs quantités de partenaire de couplage desdits biomolécules sur une surface sensiblement plane au moyen d'une liaison covalente ou quasi-covalente, d'interaction ionique, d'adhésion ou d'adsorption, l'élimination de partenaire de couplage non fixé à la surface, et l'application subséquente d'un échantillon contenant des biomolécules sur la surface dans des conditions permettant le couplage des partenaires de couplage avec les biomolécules, et le rinçage subséquent des biomolécules non couplées à des partenaires de couplage et d'autres composantes de l'échantillon ;
(b) le marquage des biomolécules couplées par une réaction impliquant un changement de couleur détectable dans le domaine visible du spectre ; et
(c) l'enregistrement des biomolécules couplées au moyen d'une image dans un système de détection optique,
**caractérisé en ce que**
la surface sensiblement plane est disposée dans un cadre ayant les dimensions d'un cadre de diapositive conventionnel de 50 mm sur 50 mm et que l'enregistrement des biomolécules couplées est réalisé par le système optique sous forme d'enregistrement électronique d'un scanner de diapositive.

2. Procédé selon la revendication 1, dans lequel le marquage des biomolécules couplées est réalisé au moyen d'une réaction impliquant un changement de réaction détectable dans le domaine visible du spectre en ce que, au choix :
(a) dans une première étape, l'on met la surface en contact avec une enzyme susceptible de réagir avec un colorant et possédant une propriété de fixation spécifique vis-à-vis des biomolécules couplées puis, dans une seconde étape, l'on met la surface en contact avec une solution d'un colorant susceptible d'être transformé par l'enzyme, de manière à ce que se déroule une réaction impliquant un changement de couleur aux sites où des biomolécules fixées à leur enzyme sont couplées à un partenaire de couplage, ou
(b) un colorant actif en fluorescence ou en luminescence lié à la biomolécule est amené à émettre une lumière par activation optique ou chimique appropriée, ou encore
(c) une particule métallique liée à la biomolécule est utilisée comme germe de cristallisation pour un traitement subséquent avec un sel de métal noble, de préférence un sel d'argent, dans des conditions réductrices, de manière à former un précipité métallique visible dans la lumière visible.

3. Dispositif comportant :
(a) un porte-échantillon pour des biomolécules, comprenant une surface essentiellement plane, sur laquelle sont couplées des partenaires de couplage pour biomolécules au moyen d'interactions covalentes, quasi-covalentes, ioniques, d'adhésion ou d'adsorption, le porte-échantillon étant disposé dans un cadre ayant les dimensions d'un cadre de diapositive conventionnel de 50 mm sur 50 mm, et
(b) un scanner de diapositive capable d'une détection électronique à la lumière visible d'une image de la surface d'un porte-échantillon introduit dans celui-ci.

4. Porte-échantillon pour biomolécules, comprenant une surface essentiellement plane, sur laquelle sont fixées des partenaires de couplage pour biomolécules au moyen d'interactions covalentes, quasi-covalentes, ioniques, d'adhésion ou d'adsorption, le porte-échantillon étant disposé dans un cadre ayant les dimensions d'un cadre de diapositive conventionnel de 50 mm sur 50 mm.
